(19) Europäisches Patentamt
European Patent Office
Office européen des brevets

(11) Veröffentlichungsnummer: **0 021 220**
**B1**

# (12) EUROPÄISCHE PATENTSCHRIFT

(45) Veröffentlichungstag der Patentschrift: **02.03.83**

(51) Int. Cl.³: **B 01 J 19/08**, C 07 C 139/04 // B01F3/04

(21) Anmeldenummer: **80103204.6**

(22) Anmeldetag: **10.06.80**

## (54) Verwendung einer Begasungsdüse bei photochemischen Gas-Flüssig-Reaktionen.

(30) Priorität: **16.06.79 DE 2924427**

(43) Veröffentlichungstag der Anmeldung: **07.01.81 Patentblatt 81/1**

(45) Bekanntmachung des Hinweises auf die Patenterteilung: **02.03.83 Patentblatt 83/9**

(84) Benannte Vertragsstaaten: **AT BE CH DE FR GB IT LI NL SE**

(56) Entgegenhaltungen:
**FR-A-2 167 782**
**FR-A-2 293 968**
**GB-A-1 164 448**
**US-A-3 658 671**
**US-A-3 829 070**

(73) Patentinhaber: **HOECHST AKTIENGESELLSCHAFT, Postfach 80 03 20, D-6230 Frankfurt/Main 80 (DE)**

(72) Erfinder: **Ramloch, Herbert, Dr., Eichhornweg 3, D-6232 Bad Soden am Taunus (DE)**
Erfinder: **Schadow, Ernst, Dr., Taubenberg 100, D-6270 Idstein/Taunus (DE)**
Erfinder: **Marx, Raimund, Wilhelm-Kalle-Strasse 16, D-6200 Wiesbaden (DE)**

EP 0 021 220 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verwendung einer Begasungsdüse bei photochemischen Gas-Flüssig-Reaktionen

Gegenstand der Erfindung ist die Verwendung einer speziellen Ausführungsform einer Begasungsdüse bei photochemischen Gas-Flüssig-Reaktionen, insbesondere beim Sulfoxidationsverfahren zur Herstellung von sekundären Alkansulfonsäuren aus geradkettigen Paraffin-Kohlenwasserstoffen in Gegenwart von Wasser durch Einstrahlen von UV-Licht.

Aus der Reaktionssummengleichung des Sulfoxidationsverfahrens

$$RH + 2SO_2 + O_2 + H_2O \xrightarrow{UV} R{-}SO_3H + H_2SO_4$$

$$R = \text{Alkyl}$$

ist ersichtlich, daß 4 Reaktionskomponenten, nämlich n-Paraffine, Wasser, Schwefeldioxid und Sauerstoff an dieser Reaktion beteiligt sind. Bei der bevorzugten Reaktionstemperatur von 30–40°C sind die n-Paraffinkohlenwasserstoffgemische und Wasser flüssig, Schwefeldioxid und Sauerstoff sind gasförmig. Das Reaktionsgefäß zur Durchführung der Sulfoxidation muß also ein Gasblasen-Flüssigkeits-Reaktor sein. Um zu guten Umsetzungen zu kommen, ist es wichtig, eine feine Verteilung des Gases in der Flüssigkeit herbeizuführen und durch besondere Strömungsverhältnisse für eine gute Vermischung der Reaktionskomponenten in der Reaktionszone und Abtransport der Reaktionsprodukte aus der Reaktionszone zu sorgen.

Das Sulfoxidationsverfahren nach obiger Reaktionsgleichung läuft aber nur dann ab, wenn UV-Licht eingestrahlt wird. Bei dieser photochemischen Gas-Flüssig-Reaktion muß also dafür gesorgt werden, daß das über eine Quecksilber-Hochdruck-Quarzglas-Tauchlampe eingestrahlte UV-Licht optimal genutzt wird. Durch das Lambertsche Gesetz ist bekannt, daß die Strahlung in der Reaktionslösung nicht linear mit der Entfernung abnimmt, sondern eine exponentielle Schwächung erfährt. Weiterhin ist bekannt, daß die Eindringtiefe der Strahlung abhängig ist von der Wellenlänge und zwar in der Weise, daß die Reichweite des UV-Lichtes der Tauchlampe von der längerwelligen Strahlung hin zur kürzerwelligen Strahlung abnimmt. Es ist also besonders wichtig, im Bereich der größten Bestrahlungsdichte, d. h. in der Nähe der Außenfläche der Tauchlampe, einen besonders guten Stoffaustausch zu erreichen.

Bei allen bekannten Verfahren der Sulfoxidation führt man die Flüssigkeit und das Gas im Kreis. Dabei kann man zwei verschiedene Arten der Stoffzuführung in den Reaktor und deren Vermischung unterscheiden.

Nach der einen Verfahrensweise wird das $SO_2-O_2$-Gasgemisch und das Paraffin-Wasser-Gemisch dem Reaktor getrennt zugeführt. Das Gasgemisch wird dabei mit Hilfe einer Frittenplatte oder einer ring- oder tellerförmigen Gasbrause, die jeweils unterhalb der UV-Tauchlampe angebracht ist, der Reaktionszone in verteilter Form zugeführt. Die Bewegung innerhalb des Reaktors wird durch die jeweilige Geschwindigkeit mit der die Kreisflüssigkeit und das Kreisgas durch den Reaktor geführt werden erzeugt. Die Geschwindigkeit der Flüssigkeit ist vor allem durch die erforderliche Verweilzeit in den nachgeschalteten Abscheidern zur Gewinnung der wäßrigen Unterphase, in der die erzeugten Alkansulfonsäuren und die Schwefelsäure enthalten sind, bestimmt. Die Geschwindigkeit des Gases ist abhängig vom Gasvordruck. Besonders bei der Verwendung von porösen Platten ist aber bei diesem Verfahren ein erheblicher Strömungswiderstand bereits innerhalb dieser Platten zu überwinden.

Nach einer anderen Verfahrensweise spritzt man die Reaktionskomponenten mit Hilfe eines oder mehrerer Hydroinjektoren in den Reaktor ein (DE-A-2 444 895). Entsprechend dem Vordruck der Flüssigkeit wird das Gas durch die Scherkräfte der Flüssigkeit fein verteilt und dem gesamten Reaktorinhalt eine Bewegung aufgezwungen. Diese Bewegung kann entsprechend der Anordnung der bzw. des Injektors in Längsrichtung zur UV-Tauchlampe verlaufen oder die Reaktionskomponenten werden radial um die Lampe geführt. Die höchste Ausbeute wurde dabei mit 3 Injektoren erzielt, die längs der Vertikalwandung eines Einlampenreaktors in regelmäßigen Abständen voneinander angebracht sind, also eine Eindüsung in tangentialer Richtung bewirken und dabei dem gesamten Reaktorinhalt eine Radialbewegung um die Lampe herum aufzwingen. Bei der Verwendung von nur einem Hydroinjektor in einem Einlampenreaktor sinkt die Ausbeute auf unter 50% der Ausbeute, die für einen Einlampenreaktor mit 3 Injektoren angegeben wird. Es ist offensichtlich, daß dieses System bei Viellampenreaktoren, wie sie zur Produktion von Alkansulfonsäure im industriellen Maßstab Verwendung finden, nut mit erheblichem technischem Aufwand angewendet werden kann.

Es wurde nun gefunden, daß die Begasung und die Vermischung der Gasphase mit der Flüssigkeit bei derartigen photochemischen Gas-Flüssig-Reaktionen im Vergleich zu den oben erwähnten vorbekannten Verfahren vereinfacht und verbessert werden kann, wenn man eine Begasungsdüse verwendet, die aus einem Gaseinleitungsrohr (1) und einem aufgesetzten Mischbecher (2) besteht, in dessen Boden 3 bis 12 Ansaugöffnungen (3) für die Flüssigphase vorhanden sind.

Im einzelnen ist diese Begasungsdüse in der Zeichnung näher dargestellt. Die Zeichnung zeigt die

Begasungsdüse im Längsschnitt mit hier 4 Ansaugöffnungen (3). Diese Ansaugöffnungen, die rund sind, haben einen Durchmesser, der etwa dem 1/4 bis 2fachen inneren Durchmesser des Gasleitungsrohres (1) entspricht. Der Winkel zwischen den Ansaugöffnungen und dem Gaseinleitungsrohr beträgt 30° bis 90°. Der Innendurchmesser des Mischbechers (2) beträgt etwa 1/8 bis 1/2 des Durchmessers des äußeren Hüllrohrs der UV-Tauchlampe. Das Verhältnis von Höhe und innerem Durchmesser des Mischbechers beträgt ca. 1 : 1 bis 3 : 1. Der innere Querschnitt des Gaseinleitungsrohres (1) ist kreisrund bis sternförmig ausgestaltet. Die Länge des Gaseinleitungsrohrs ist unkritisch, doch muß das Gaseinleitungsrohr so lang sein, daß ein ausreichender Abstand der Ansaugöffnungen vom Reaktorboden oder — bei einem Viellampenreaktor — von einem gemeinsamen Gaszuführungsrohr gewährleistet ist, damit die Flüssigphase vor dem Gasstrom ungehindert durch die Ansaugöffnungen angesaugt werden kann. Als Material, aus dem diese Begasungsdüsen bestehen, eignet sich beim Einsatz bei der Sulfoxidation besonders glasfaserverstärktes Polytetrafluorethylen.

Diese Begasungsdüsen werden in dem Reaktor unterhalb der UV-Tauchlampe montiert und zwar pro Tauchlampe eine Düse, wobei diese Düsen in einem Viellampenreaktor pro Tauchlampenreihe jeweils auf einem gemeinsamen Gaszuführungsrohr montiert sein können. Die Begasungsdichte für eine ausreichende Gasfeinverteilung und besonders günstige Strömungsverhältnisse entlang der Längsrichtung der Lampe beträgt etwa 50 — 500 vorzugsweise 200 — 500 $m^3$ pro $m^2$ sec. Zu beachten ist dabei, daß der Vordruck des Gases auch vom Flüssigkeitsstand über der Düse abhängig ist.

Das Gasgemisch tritt mit hoher Geschwindigkeit aus dem engen Teil der Düse in die trichterförmige Erweiterung aus, dadurch wird dort durch die seitlichen Öffnungen Flüssigkeit angesaugt. Durch die Scherkräfte der angesaugten Flüssigkeit wird das Gasgemisch fein verteilt. Den Trichter der Düse verläßt ein Flüssigkeits-Gas-Strahl, der der gesamten Reaktionsflüssigkeit innerhalb des Reaktors eine Bewegung nach dem Prinzip einer Mammutpumpe aufzwingt. Durch die Bauweise dieser Begasungsdüse ist gewährleistet, daß der Stoffaustausch im Nahbereich des äußeren Lampenhüllrohrs, also im Bereich der größten Bestrahlungsdichte, am besten ist und daß in diesem Bereich die Konzentration der Gasblasen am größten ist und die Geschwindigkeit des Stofftransports am höchsten ist. Man erhält mit dieser Begasungsdüse auf einfache Weise eine hohe Ausbeute an Alkansulfonsäuren bedingt durch eine verbesserte Nutzung des eingestrahlten UV-Lichts und durch verbesserte Stoffübergänge. Dies gilt sowohl bei der Anwendung in einem Einlampenreaktor als auch in einem Viellampenreaktor.

Die beschriebene Begasungsdüse eignet sich für alle Gas-Flüssig-Reaktionen, vorzugsweise aber für die Herstellung sek.-Alkansulfonate durch Sulfoxidation. Hierbei werden n-Paraffinkohlenwasserstoffe oder deren Gemische mit 8 — 30 C-Atomen, bevorzugt 10 — 18 C-Atomen, eingesetzt, sowie ein $SO_2 - O_2$-Gasgemisch in einem molaren Verhältnis von ca. 2 : 1, also dem stöchiometrischen Verhältnis, oder vorzugsweise mit einem $SO_2$-Überschuß bis zu einem Molverhältnis von etwa 3 : 1. Die Reaktionstemperatur betägt 20 — 40° C, vorzugsweise 30 — 38° C. Im Reaktor wird ein Druck bis etwa 1 barü, vorzugsweise 0,08 bis 0,25 barü, gehalten. Die Reaktionsflüssigkeit wird am Reaktorboden entnommen, über die Abscheideeinrichtung geführt und dort die Unterphase, die die produzierten Alkansulfonsäuren und die Schwefelsäure neben Paraffin und Wasser enthält, abgetrennt. Die Oberphase, die Paraffinphase, wird gekühlt und dann nach Auffrischung mit Paraffin und Wasser am Kopf des Reaktors wieder zurückgeführt. Die Entnahme des ebenfalls im Kreis geführten $SO_2 - O_2$-Gasgemisches erfolgt aus der Gaszone im oberen Bereich des Reaktors. Nach der Auffrischung mit $SO_2$ und $O_2$ wird das Gasgemisch in einem Kompressor auf den gewünschten Vordruck komprimiert und über die beschriebene Begasungsdüse in den Reaktor eingebracht.

## Beispiel

Das verwendete Reaktionsgemisch war ein handelsüblicher Schnitt aus n-Paraffinen mit 12 bis 18 C-Atomen. 4 $m^3$/h Flüssigkeit wurden unten aus dem Reaktor entnommen, über das Abscheidesystem geführt und, nach Auffrischung mit Paraffin und Wasser, über den Kopf des Reaktors zurückgepumpt. Der Reaktor war zu ca. 5/6 seines Volumens mit Flüssigkeit und zu ca. 1/6 darüber mit Gas gefüllt. Der Druck im Gasraum des Reaktoroberteils betrug 0,13 bar. Die Reaktionstemperatur ist durch Kühlen der Kreisflüssigkeit bei 31 — 33° C gehalten worden. Die Einstrahlung von UV-Licht erfolgte durch eine Quecksilber-Hochdruck-Tauchlampe mit einer Stromaufnahme von 3,84 KW. Der Durchmesser ihres äußeren Quarzhüllenrohres betrug 120 mm. Diese UV-Lampe erzeugte einen Strahlungsfluß von 545 Watt im Bereich der Wellenlängen von 248 bis 400 nm. 33 $m^3$/h $SO_2 - O_2$-Gasgemisch einer molaren Zusammensetzung von 2,2 : 1 wurde durch eine Begasungsdüse wie oben beschrieben mit einem Vordruck von 1,1 bar in den Reaktor eingesprüht. Diese Vorrichtung war im unteren Reaktorboden, genau unterhalb der UV-Lampe, befestigt und hatte folgende Abmessungen: Die Gasaustrittsöffnung hatte eine lichte Weite von 7 mm, die symmetrisch angeordneten 4 Ansaugöffnungen eine lichte Weite von jeweils 10 mm, der Innendurchmesser des Mischbechers betrug 35 mm, die Begasungsdüse war insgesamt 110 mm und der Mischbecher ab Gasaustrittsöffnung 50 mm hoch. Pro Stunde wurden so 76 Alkansulfonsäure-Äquivalente erzeugt. Das molare Verhältnis von Mono- zu Disulfonsäure lag

bei ca. 9 : 1.

Arbeitet man in dem gleichen Reaktor und unter den gleichen Bedingungen, wie oben angegeben, jedoch mit einer bei der Sulfoxidation bisher benutzten Lochbrause mit einem Durchmesser von 180 mm, die auf ihrer Oberfläche Bohrungen mit einer Lochweite von ca. 0,8 bis 1,5 mm aufweist und führt durch diese Lochbrause 75 m³/h eines $SO_2-O_2$-Gasgemisches der molaren Zusammensetzung 2,2 : 1 in den Reaktor ein, so erhält man pro Stunde nur 52 Alkansulfonsäure-Äquivalente.

**Patentansprüche**

1. Verwendung einer Begasungsdüse, bestehend aus einem Gaseinleitungsrohr (1) und einem aufgesetzten Mischbecher (2), in dessen Boden 3 bis 12 Ansaugöffnungen (3) für die Flüssigkeit vorhanden sind, bei photochemischen Gas-Flüssig-Reaktionen.

2. Verwendung der Begasungsdüse nach Anspruch 1 bei der Sulfoxidation von n-Paraffinen unter Einwirkung von UV-Licht.

**Claims**

1. Method of use of a gasification jet consisting of a gas inlet tube (1) and a mixing beaker at top (2) having in the bottom 3 to 12 suction orifices (3) for the liquid, in photochemical gas liquid reactions.

2. Method of use of the gasification jet according to claim 1 in the sulfoxidation of n-paraffins under radiation of UV-light.

**Revendications**

1. Utilisation d'une buse d'introduction de gaz, consistant en un tube (1) d'introduction de gaz surmonté d'une cuvette (2) de mélange, dans le fond de laquelle sont percés trois à douze orifices (3) d'aspiration du liquide, pour des réactions photochimiques gaz/liquide.

2. Utilisation de la buse d'introduction de gaz selon la revendication 1 pour la sulfoxydation de paraffines normales sous l'effet de la lumière ultraviolette.

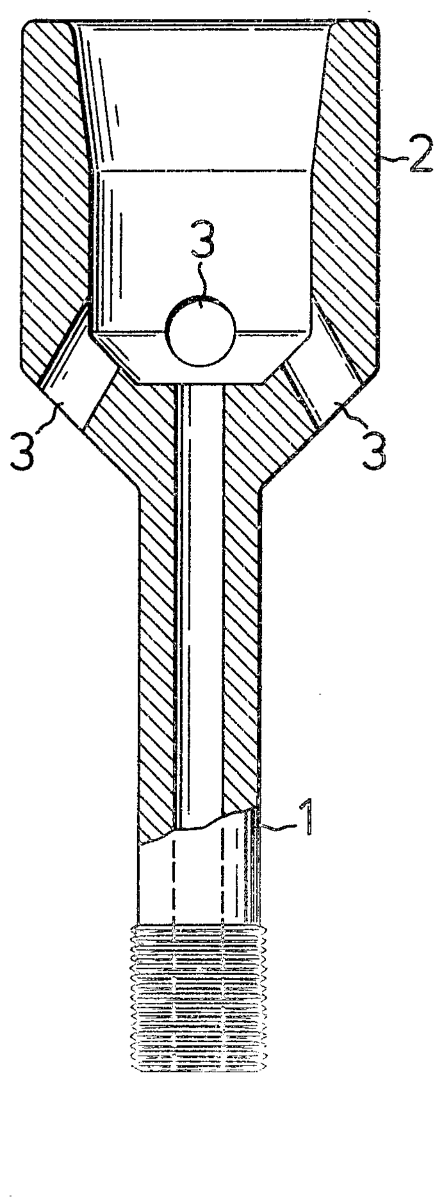
2
3
3
3
1